# EUROPEAN PATENT APPLICATION

(11) **EP 3 156 479 A1**
(43) Date of publication of application: **19.04.2017**
(21) Application number: 15806955.9
(22) Date of filing: 10.06.2015
(51) Int. Cl.: C12M 3/00, C12M 1/00

(54) **SEALED CONTAINER AND CONVEYANCE SYSTEM**

(30) Priority: 12.06.2014 JP 2014121743
(71) Applicant: TOKYO ELECTRON LIMITED, Minato-ku Tokyo 107-6325 (JP)
(72) Inventor: TAMURA Akitake, Tokyo 107-6325 (JP)
(74) Representative: Diehl & Partner GbR
(86) International application number: PCT/JP2015/066676
(87) International publication number: WO 2015/190502

(57) **Abstract**

A sealed container includes a container body having an opening, a lid removably installed in the opening of the container body, and a first seal member installed to seal a gap between the container body and the lid. A locking hole is formed in an inner peripheral portion of the container body. A first opening capable of facing the locking hole is formed in an outer peripheral portion of the lid. A second opening communicating with the first opening is formed in an end portion of the lid. The lid includes a locking member that can linearly move to protrude from and retract into the first opening, a rotatable rotary member positioned to face the second opening, a conversion mechanism configured to covert rotational motion of the rotary member to linear motion of the locking member, and a second seal member installed to seal the second opening.

## Description

### TECHNICAL FIELD

The present disclosure relates to a sealed container and, more particularly, to a sealed container capable of accommodating a plurality of cell culture containers. Furthermore, the present disclosure pertains to a conveyance system that makes use of such a sealed container.

### BACKGROUND

In recent years, the research and development of a regenerative medicine that artificially creates target tissues or organs by cell culture is underway. A cell culture operation is performed within a cell culture facility that satisfies a predetermined criterion, for example, a GMP (Good Manufacturing Practice). In such a cell culture facility, an aseptic management and an aseptic operation are required. As such a facility, for example, a CPC (Cell Processing Center) is known.

In the meantime, attempts have been made to automate cell culture to some extent. In Japanese patent laid-open publication No. 2008-206495 (hereinafter referred to as Patent Document 1), there is disclosed an aspect which includes a culture device configured to culture cells accommodated within a culture container and a carrier case used for conveying a culture container between a culture device and a clean bench. In a state in which the opening of the carrier case and the loading gate of the culture device are disposed to face each other, a door that closes the loading gate is opened. Then, by operating a conveyance arm existing within the culture device, the culture container is loaded from the carrier case into the culture device.

However, in Patent document 1, nothing is described on a structure like a lid for closing the opening of the carrier case. Furthermore, nothing is described on a structure for sealing a gap between the culture device and the carrier case when the door of the culture device is opened. Therefore, in order to maintain the aseptic state of the interior of the carrier case and the interior of the culture device at the time of loading the culture container, it is necessary to aseptically manage the external space of the culture device and the carrier case. In a site that handles cells, such as iPS cells or the like, which are supposed to eventually return to a human body or the like, aseptic management becomes very burdensome and equipment cost, maintenance cost or the like becomes expensive.

In Japanese utility model laid-open publication No. sho 51-49520 (hereinafter referred to as Patent Document 2) and Japanese patent laid-open publication No. hei 6-193323 (hereinafter referred to as Patent Document 3), there is disclosed a sealed container for accommodating a hazardous material such as a radioactive material, a toxic material or the like in a sealed state, the sealed container including a container body with an opening and a lid detachably installed in the opening of the container body in a bayonet manner.

However, in the sealed container disclosed in Patent document 2 and Patent document 3, a bayonet type mechanism is employed as a lid attachment/detachment mechanism. Thus, when attaching and detaching the lid, it is necessary to perform an operation of rotating the lid with respect to the container body. This makes it difficult to realize automation. Furthermore, since the bayonet type mechanism is employed as the lid attachment/detachment mechanism, a sliding part exists between the lid and the container body. Inasmuch as dust is generated from the sliding part at the time of attaching and detaching the lid, it is impossible to maintain the cleanliness of the interior of the container body at a high level.

In Japanese patent laid-open publication No. 2010-3948 (hereinafter referred to as Patent Document 4) and Japanese patent laid-open publication No. 2003-174080 (hereinafter referred to as Patent Document 5), there is disclosed a storage container for storing semiconductor wafers, the storage container including a container body with an opening and a lid detachably installed in the opening of the container body. In this storage container, a rotary member and a locking member connected to the rotary member via a crank mechanism are disposed within the lid. In the opposite end portion of the lid from the container body, an operation pin insertion hole is formed so as to correspond to the rotary member. An operation pin is inserted into the lid from the operation pin insertion hole and is brought into engagement with the rotary member. As the operation pin rotates together with the rotary member, the locking member protrudes radially outward due to the operation of the crank mechanism. The locking member is inserted into a locking hole formed in the container body. Thus, the lid is fixed with respect to the container body.

In the storage container disclosed in Patent Document 4 and Patent Document 5, it is not necessary to perform an operation of rotating the lid with respect to the container body at the time of attaching or detaching the lid. Thus, it is easy to realize automation of the operation performed at the time of attaching or detaching the lid and it is possible to prevent generation of dust from the sliding part at the time of attaching or detaching the lid.

The present inventor initially considered to use such a storage container for storing semiconductor wafers as a closed container for accommodating cell culture containers. However, in the storage container for storing semiconductor wafers, since the operation pin insertion hole is formed in the opposite end portion of the lid from the container body, there is a possibility that a gas existing outside the lid enters the interior of the storage container through the operation pin insertion hole. This poses a problem in that it is impossible to aseptically manage the interior of the storage container.

By paying attention to the above problems, the present disclosure has been made to effectively solve the problems. It is an object of the present disclosure to provide a sealed container capable of preventing a gas from entering the interior of a container from the outside of a lid.

### SUMMARY

According to one embodiment of the present disclosure, there is provided a sealed container, including: a container body having an opening; a lid removably installed in the opening of the container body; and a first seal member installed so as to seal a gap between the container body and the lid, wherein a locking hole is formed in an inner peripheral portion that defines the opening of the container body, a first opening capable of facing the locking hole is formed in an outer peripheral portion of the lid, a second opening communicating with the first opening is formed in an end portion of the lid opposite to the container body, and the lid includes a locking member capable of linearly moving in such a direction as to protrude from and retract into the first opening, a rotatable rotary member positioned to face the second opening, a conversion mechanism configured to covert rotational motion of the rotary member to linear motion of the locking member, and a second seal member installed so as to seal the second opening.

Preferably, the second seal member is installed in a gap between an inner peripheral portion that defines the second opening and the rotary member, and at least a part of an end portion of the rotary member opposite to the container body is exposed to the outside.

In this case, specifically, for example, the second seal member is one of a magnetic fluid seal, an oil seal, an O-ring seal and a bellows seal.

Alternatively, the second seal member is installed at an opposite side of the rotary member from the container body so as to cover the rotary member.

In this case, specifically, for example, the second seal member is a magnetic coupling seal.

According to another embodiment of the present disclosure, there is provided a sealed container, including: a container body having an opening; a lid removably installed in the opening of the container body; and a first seal member installed so as to seal a gap between the container body and the lid, wherein the container body includes a step portion capable of facing an edge of a container-body-side end portion of the lid and a metal body installed in the step portion, a second opening is formed in an end portion of the lid opposite to the container body, and the lid includes a magnet installed so as to correspond to the metal body of the step portion, a yoke member capable of linearly moving in such a direction that the yoke member is inserted into and removed from between the magnet and the metal body, a rotatable rotary member positioned to face the second opening, a conversion mechanism configured to covert rotational motion of the rotary member to linear motion of the yoke member, and a second seal member installed so as to seal the second opening.

According to a further embodiment of the present disclosure, there is provided a sealed container, including: a container body having an opening; a lid removably installed in the opening of the container body; and a first seal member installed so as to seal a gap between the container body and the lid, wherein the container body includes a step portion capable of facing an edge of a container-body-side end portion of the lid and a permanent magnet or a magnetic body installed in the step portion, and a permanent electromagnet configured to change a magnetic force as a pulsed magnetic field is applied thereto is installed in the lid so as to correspond to the permanent magnet or the magnetic body of the step portion.

In this case, specifically, for example, the permanent electromagnet is a samarium-cobalt magnet or an iron-cobalt-vanadium soft magnetic alloy.

According to a still further embodiment of the present disclosure, there is provided a sealed container, including: a container body having an opening; a lid removably installed in the opening of the container body; and a first seal member installed so as to seal a gap between the container body and the lid, wherein a locking member configured to lock the lid to the container body, a movable member configured to move the locking member, and a second seal member configured to seal a gap between the lid and the movable member, are installed within the lid.

In the sealed container having any one of the features described above, the first seal member may include a first seal element installed in an edge of an inner peripheral portion that defines the opening of the container body.

In this case, the first seal element may be an O-ring seal having a substantially triangular cross section.

In the sealed container having any one of the features described above, the first seal member may include a second seal element installed in a gap between a step portion capable of facing an edge of a container-body-side end portion of the lid and the edge of the container-body-side end portion of the lid.

In the sealed container having any one of the features described above, a shelf capable of accommodating a plurality of cell culture containers may be installed inside the container body.

According to a yet still further embodiment of the present disclosure, there is provided a conveyance system, including: the sealed container having any one of the features described above; a conveyance part configured to convey the sealed container; and a receiving part configured to receive the sealed container conveyed by the conveyance part, wherein the receiving part includes a receiving part body having an opening, a door removably installed in the opening of the receiving part body, and a third seal member installed in an edge of the door so as to seal a gap between the receiving part body and the door, the first seal member includes a first seal element installed in an edge of an inner peripheral portion that defines the opening of the container body, and when the sealed container and the receiving part are disposed in such an orientation that the openings of the sealed container and the receiving part face each other, the first seal element is brought into close contact with the receiving part body to seal a gap between the container body and the receiving part body, and the third seal member is brought into close contact with the lid to seal a gap between the door and the lid.

### BRIEF DESCRIPTION OF THE DRAWINGS

FIG. 1 is a schematic side sectional view illustrating a sealed container according to a first embodiment of the present disclosure.
FIG. 2 is a schematic side sectional view illustrating a state in which an operation pin is brought into engagement with an operation pin engagement hole of a rotary member in the sealed container of **FIG. 1****.**
**FIG. 3** is a schematic side sectional view illustrating a state in which a locking member is removed from a locking hole in the sealed container of **FIG. 1****.**
**FIG. 4** is a schematic side sectional view illustrating a state in which a lid is removed from a container body in the sealed container of **FIG. 1****.**
**FIG. 5** is a schematic plane view illustrating one example of cell culture containers accommodated within the sealed container of **FIG. 1****.**
**FIG. 6A** is a schematic plane view for explaining the operations of a locking member and a rotary member in the sealed container of **FIG. 1****.**
**FIG. 6B** is a schematic plane view for explaining the operations of the locking member and the rotary member in the sealed container of **FIG. 1****.**
**FIG. 7** is a schematic side sectional view illustrating an aspect in which a container body is laterally opened and in which a shelf is installed at the side of the lid.
**FIG. 8** is a schematic side sectional view illustrating an aspect in which the container body is laterally opened and in which the shelf is installed at the side of the container body.
**FIG. 9** is a schematic view for explaining a second example of a second seal member.
**FIG. 10** is a schematic view for explaining a third example of the second seal member.
**FIG. 11** is a schematic plane view illustrating a configuration of a conveyance system which makes use of the sealed container of **FIG. 1****.**
**FIG. 12** is a schematic side view illustrating, on an enlarged scale, a conveyance part of the conveyance system of **FIG. 11** and an automatic culture device.
**FIG. 13** is a control block diagram illustrating a control mode of the automatic culture device of **FIG. 11****.**
**FIG. 14** is a schematic side sectional view illustrating a sealed container according to a second embodiment of the present disclosure.
**FIG. 15A** is a schematic view for explaining a magnetic circuit in the sealed container of **FIG. 14****.**
**FIG. 15B** is a schematic view for explaining the magnetic circuit in the sealed container of **FIG. 14****.**
**FIG. 16** is a schematic side sectional view illustrating a sealed container according to a third embodiment of the present disclosure.

### DETAILED DESCRIPTION

Embodiments of the present disclosure will now be described in detail with reference to the accompanying drawings. The present disclosure disclosed herein is not limited by the embodiments. The respective embodiments may be appropriately combined unless inconsistent with the processing contents.

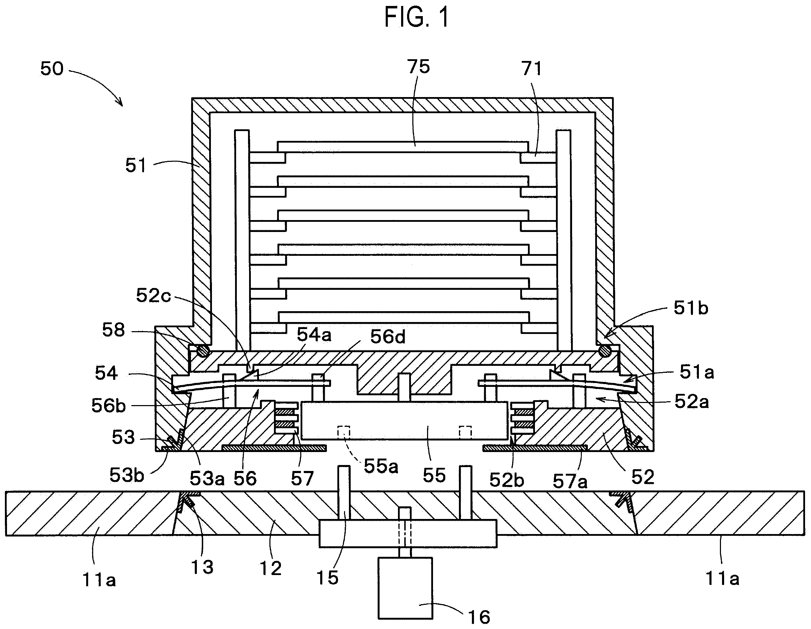

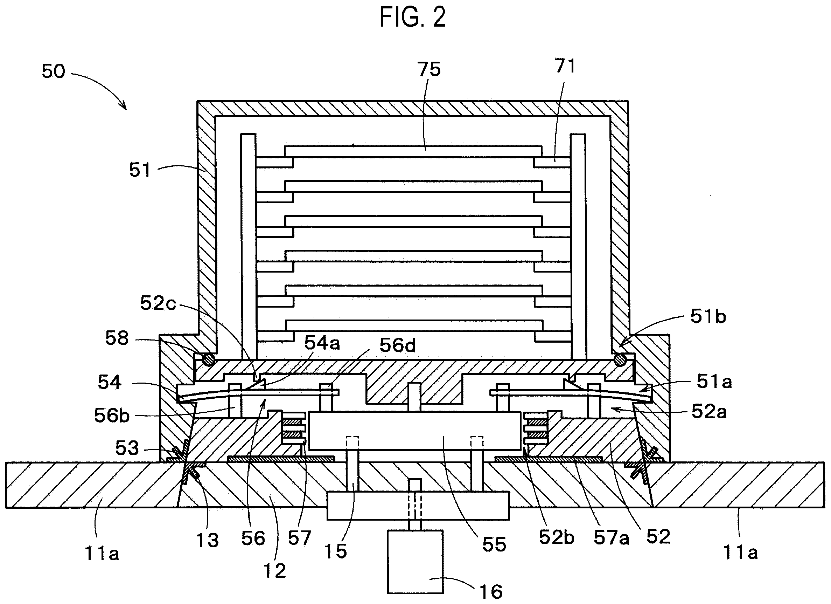

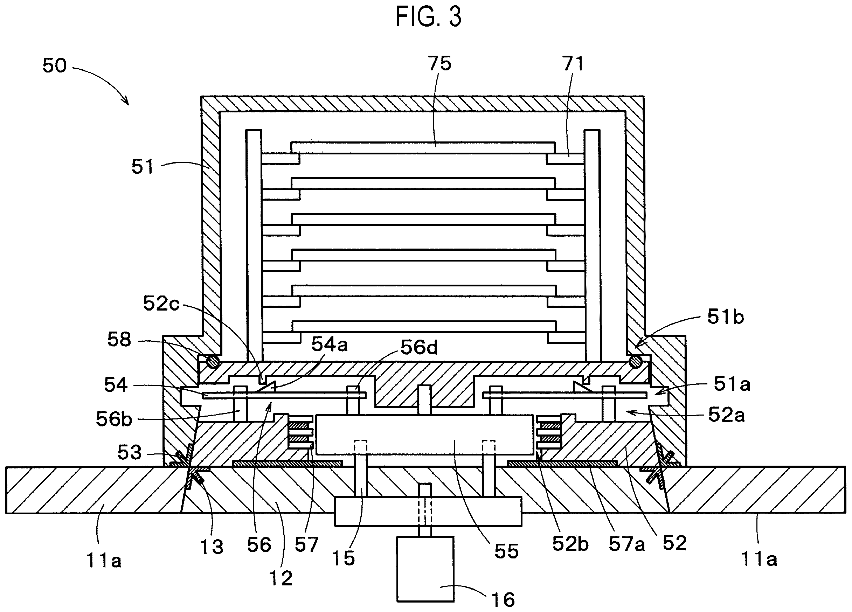

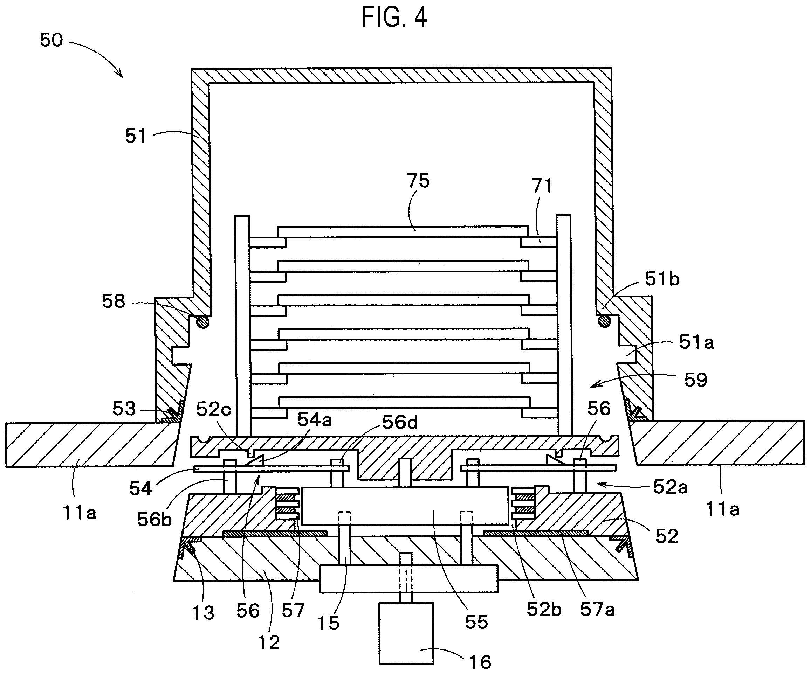

**FIG. 1** is a schematic side sectional view illustrating a sealed container according to a first embodiment of the present disclosure. **FIG. 2** is a schematic side sectional view illustrating a state in which an operation pin is brought into engagement with an operation pin engagement hole of a rotary member in the sealed container of **FIG. 1****.** **FIG. 3** is a schematic side sectional view illustrating a state in which a locking member is removed from a locking hole in the sealed container of **FIG. 1****.** **FIG. 4** is a schematic side sectional view illustrating a state in which a lid is removed from a container body in the sealed container of **FIG. 1****.**

The sealed container according to the present embodiment is a sealed container used to accommodate cell culture containers for culturing cells or the like in a sealed state, in a facility for automatically culturing different cells including pluripotent stem cells such as iPS cells, ES cells or the like, cartilage cells such as bone marrow stromal cells (MSC) or the like, and dendritic cells. In the present embodiment, an aspect in which iPS cells are used will be mainly described herein below. However, this is nothing more than one example.

As illustrated in FIGS. 1 to 4, the sealed container 50 according to the present embodiment includes a container body 51 having an opening 59, a lid 52 detachably installed in the opening 59 of the container body 51, and a first seal member installed so as to seal a gap between the container body 51 and the lid 52.

Among them, the container body 51 is formed in a cylindrical shape with a bottom and is made of a resin so that the container body 51 includes a cylindrical sidewall and a bottom portion configured to close one end of the sidewall. The opening 59 is oriented to face downward. In order to facilitate insertion and removal of the lid 52, the inner peripheral portion that defines the opening 59 of the container body 51 is formed so as to have a tapered shape in which the opening area grows larger toward the lower end portion. Similarly, in order to facilitate insertion and removal of the lid 52, the side surface of the lid 52 is formed so as to have a tapered shape in which the cross-sectional area grows smaller toward the upper end portion. In the present embodiment, each of the inner peripheral portions that define the opening 59 of the container body 51 and the lid 52 includes a contour having a circular plan-view shape. However, the present disclosure is not limited thereto. Each of the inner peripheral portion and the lid 52 may include a contour having an elliptical or polygonal plan-view shape.

The first seal member is formed of an elastic member (O-ring seal) having an annular plan-view shape. The first seal member includes a first seal element 53 tightly fixed to an edge of the inner peripheral portion that defines the opening 59 of the container body 51. In the present embodiment, the first seal element 53 has a cross section of a substantially triangular shape (preferably, an obtuse-angled triangular shape). More specifically, the first seal element 53 includes a first surface 53a (an inner circumferential surface) and a second surface 53b (a bottom surface) inclined by a predetermined obtuse angle with respect to the first surface 53a. The first surface 53a of the first seal element 53 is positioned so as to protrude more radially inward than the inner circumferential surface that defines the opening 59 of the container body 51. The second surface 53b of the first seal element 53 is positioned so as to protrude toward the lid 52 (downward) beyond the lid-side end portion (lower end portion) of the container body 51. The term "substantially triangular shape" used herein refers to a shape including at least a first side forming a generatrix of the first surface (inner circumferential surface) and a second side forming a generatrix of the second surface (bottom surface). As long as the "substantially triangular shape" has at least a first side and a second side, the "substantially triangular shape" may be an arrow shape as a whole as illustrated in **FIGS. 1** to **4** or may be a triangle shape, a square shape or a fan shape as a whole while not shown in the drawings.

As illustrated in **FIGS. 1** to **3****,** when the lid 52 is inserted into the opening 59 of the container body 51, the side surface of the lid 52 is brought into close contact with the first surface 53a (the inner circumferential surface) of the first seal element 53 without making contact with the inner surface of the container body 51. Thus, the gap between the lid 52 and the container body 51 is sealed by the first seal element 53.

As illustrated in **FIGS. 1** to **4****,** a locking hole 51a is formed in the inner peripheral portion that defines the opening 59 of the container body 51. A first opening 52a capable of facing the locking hole 51a is formed in the outer peripheral portion of the lid 52. Furthermore, a second opening 52b communicating with the first opening 52a is formed in the end portion (lower end portion) of the lid 52 opposite to the container body 51.

Furthermore, the lid 52 includes a pair of locking members 54 that can linearly move in such a direction as to protrude from and retract into the first opening 52a, a rotatable rotary member 55 positioned to face the second opening 52b, a conversion mechanism 56 configured to convert rotational motion of the rotary member 55 into linear motion of the locking member 54, and a second seal member 57 installed so as to seal the second opening 52b.

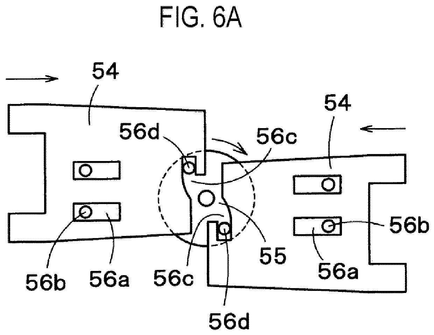

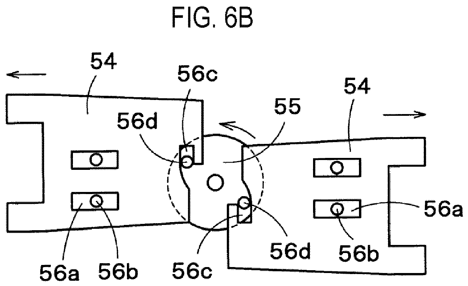

As illustrated in **FIGS. 6A** and **6B****,** the rotary member 55 has a circular plan-view shape. As illustrated in **FIGS. 1** to **4****,** the rotary member 55 is rotatably supported in the container-body-side end portion of the lid 52 on the container body 51. An operation pin fitting hole 55a is formed in the end portion of the rotary member 55 opposite to the container body 51.

**FIGS. 6A** and **6B** are schematic plane view for explaining the operations of the locking members 54 and the rotary member 55.

As illustrated in **FIGS. 6A** and **6B****,** the locking members 54 are disposed at the opposite sides with respect to the rotation axis of the rotary member 55. Two first grooves 56a having a linear shape are formed in each of the locking members 54 so as to extend parallel to the direction in which the locking members 54 protrude from or retract into the first opening 52a. A first guide member 56b is inserted into each of the first grooves 56a. Furthermore, a second groove 56c having an arc shape is formed in the base end portion of each of the locking members 54. A second guide member 56d extending parallel to the rotation axis of the rotary member 55 is inserted into the second groove 56c. As illustrated in **FIGS. 1** to **4****,** the first guide member 56b is fixed to the end portion of the lid 52 opposite to the container body 51. The second guide member 56d is fixed to the rotary member 55.

As illustrated in **FIG. 6B****,** if the rotary member 55 is rotated counterclockwise, the second guide member 56d is guided along the second groove 56c while pushing radially outward the wall surface that defines the second groove 56c. At this time, the first guide member 56b is guided along each of the first grooves 56a. Thus, the movement direction of the locking members 54 is restricted to a direction parallel to the first grooves 56a, namely a direction in which the locking members 54 protrude from the first opening 52a. Consequently, the rotational motion of the rotary member 55 is converted to the linear motion of the locking members 54. The locking members 54 are linearly moved in a direction in which they move away from each other.

On the other hand, if the rotary member 55 is rotated clockwise as illustrated in **FIG. 6A**, the second guide member 56d is guided along the second groove 56c while pushing radially inward the wall surface that defines the second groove 56c. At this time, the first guide member 56b is guided along each of the first grooves 56a. Thus, the movement direction of the locking members 54 is restricted to a direction parallel to the first grooves 56a, namely a direction in which the locking members 54 retracted into the first opening 52a. Consequently, the rotational motion of the rotary member 55 is converted to the linear motion of the locking members 54. The locking members 54 are linearly moved in a direction in which they move toward each other.

As described above, in the present embodiment, the conversion mechanism 56 for converting the rotational motion of the rotary member 55 to the linear motion of the locking members 54 is composed of the first grooves 56a, the first guide member 56b, the second groove 56c and the second guide member 56d.

As illustrated in **FIGS. 1** to **4****,** the second seal member 57 is installed in the gap between the inner peripheral portion that defines the second opening 52b of the lid 52 and the rotary member 55. At least a part of the end portion of the rotary member 55 opposite to the container body 51 is exposed to the outside. The operation pin fitting hole 55a described above is formed in the externally-exposed part of the rotary member 55.

In the present embodiment, a magnetic fluid seal is used as the second seal member 57. In the illustrated example, the second seal member 57 includes a ring-shaped magnet fixed to the inner peripheral portion that defines the second opening 52b, and a magnetic fluid filled in the gap between the magnet and the rotary member 55. At least a part of the rotary member 55 facing the magnet is made of a material with high permeability (for example, martensitic stainless steel SUS440C). A magnetic circuit is formed between the magnet and the rotary member 55. By the magnetic circuit, the magnetic fluid continues to be held in the gap between the magnet and the rotary member 55. Thus, while permitting rotation of the rotary member 55, it is possible to prevent a gas existing outside the lid 52 form entering the interior of the sealed container 50 through the second opening 52b. In the end portion of the lid 52 opposite to the container body 51, a ring-shaped seal cover 57d is installed so as to protect the second seal member 57 from the outside.

As illustrated in **FIGS. 1** to **4****,** an inclined member 54a having a triangular cross section is installed on the upper surface of each of the locking members 54. In the main body of the lid 52, a projection portion 52c is installed so as to correspond to the inclined member 54a. The line normal to a slant surface of the inclined member 54a has a radially outward component.

As illustrated in **FIG. 2****,** when the locking members 54 are linearly moved in such a direction as to protrude from the first opening 52a, the tip of the projection portion 52c relatively moves so as to run up along the slant surface of the inclined member 54a. At this time, the inclined member 54a is pushed downward by the projection portion 52c and the tip portion of each of the locking members 54 is bent downward. Thus, the tip portion of each of the locking members 54 inserted into the locking hole 51a pushes downward the inner surface of the container body 51 that defines the locking hole 51 a. By this push force, the lid 52 is pressed against the container body 51 and is firmly fixed to the container body 51.

On the other hand, when the locking members 54 are linearly moved in such a direction as to retract into the first opening 52a as illustrated in **FIG. 3****,** the tip of the projection portion 52c relatively moves so as to run down along the slant surface of the inclined member 54a. At this time, the downward push force of the projection portion 52c applied to the inclined member 54a is gradually reduced and the tip portion of each of the locking members 54 is not bent anymore.

As illustrated in **FIGS. 1** to **4****,** a shelf 71 capable of accommodating a plurality of cell culture containers 75 is installed within the sealed container 50. In the present embodiment, the shelf 71 is vertically installed on the container-body-side end portion (upper end portion) of the lid 52 and is capable of holding the cell culture containers 75 in a vertically overlapping state.

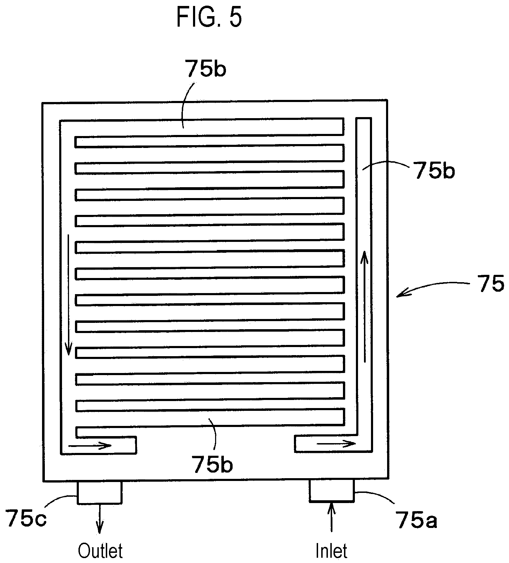

**FIG. 5** is a schematic plane view illustrating one example of the cell culture container 75 accommodated within the sealed container 50. As illustrated in **FIG. 5****,** the cell culture container 75 is a closed-type culture container (culture plate). The cell culture container 75 includes an inlet port 75a, an outlet port 75c, and a passage 75b having one end communicating with the inlet port 75a and the other end communicating with the outlet port 75c. As indicated by arrows in **FIG. 5****,** a liquid such as a liquid medium or the like flowing in from the inlet port 75a passes through the passage 75b and then flows out from the outlet port 75c. The cell culture container 75 is not limited to the closed type but may be an open-type cell culture container called a dish or a petri dish.

As illustrated in **FIGS. 1** to **4****,** the container body 51 includes a step portion 51b capable of facing the edge of the container-body-side end portion (upper end portion) of the lid 52. The first seal member includes a second seal element 58 installed between the step portion 51b and the edge of the container-body-side end portion of the lid 52.

Specifically, the second seal element 58 is, for example, an O-ring seal and is tightly fixed onto the step portion 51b. When the lid 52 is inserted into the opening 59 of the container body 51, the edge of the container-body-side end portion of the lid 52 is brought into close contact with the second seal element 58 on the step portion 51b in a ring shape without making contact with the step portion 51b of the container body 51. The gap between the edge of the container-body-side end portion of the lid 52 and the step portion 51b is sealed by the second seal element 58.

Next, the operation of the sealed container 50 of the present embodiment will be described.

First, a state in which the lid 52 is inserted into and fixed to the opening 59 of the container body 51 will be described. In this state, as illustrated in FIG. 1, the tip portion of each of the locking members 54 is inserted into the locking hole 51a of the container body 51. Thus, the lid 52 is fixed to the container body 51 and is not dropped by the gravity. Furthermore, the inclined member 54a is pushed downward by the projection portion 52c, whereby the tip portion of each of the locking members 54 is bent downward to press downward the inner surface that defines the locking hole 51 a of the container body 51. By this push force, the lid 52 is pushed toward the container body 51 and is further firmly fixed to the container body 51. A plurality of cell culture containers 75 is held by the shelf 71 vertically installed on the lid 52.

As illustrated in **FIG. 1****,** the side surface of the lid 52 is brought into close contact with the inner circumferential surface of the first seal element 53 without making contact with the inner surface of the container body 51. Thus, the gap between the lid 52 and the container body 51 is sealed by the first seal element 53. Furthermore, the edge of the container-body-side end portion of the lid 52 is brought into close contact with the second seal element 58 on the step portion 51b in a ring shape without making contact with the step portion 51b of the container body 51. Thus, the gap between the edge of the container-body-side end portion of the lid 52 and the step portion 51b is sealed by the second seal element 58. In this way, the gap between the lid 52 and the container body 51 is doubly sealed by the first seal element 53 and the second seal element 58 of the first seal member, whereby a gas existing outside the lid 52 is reliably prevented from entering the interior of the sealed container 50 through the gap between lid 52 and the container body 51. Furthermore, the gap between the inner peripheral portion that defines the second opening 52b of the lid 52 and the rotary member 55 is sealed by the second seal member 57. Thus, a gas existing outside the lid 52 is prevented from entering the interior of the sealed container 50 through the second opening 52b.

Next, a process of detaching the lid 52 from the container body 51 will be described.

First, as illustrated in **FIG. 2****,** an operation pin 15 disposed outside the lid 52 is inserted from the second opening 52b into the lid 52. The tip portion of the operation pin 15 is fitted to the operation pin fitting hole 55a of the rotary member 55.

Next, as illustrated in **FIG. 3****,** the operation pin 15 is rotationally driven by a motor 16. The rotary member 55 fitted to the tip portion of the operation pin 15 is rotated by the rotational power received from the operation pin 15. Since the operation pin 15 makes contact with the rotary member 55, the rotational power can be easily and reliably transmitted from the operation pin 15 to the rotary member 55.

The rotational motion of the rotary member 55 is converted to the linear motion of the locking members 54 by the conversion mechanism 56. Each of the locking members 54 is linearly moved in such a direction as to retract into the first opening 52a. The tip portion of each of the locking members 54 is removed from the locking hole 51 a of the container body 51. Thus, the lid 52 can be detached from the container body 51.

Next, as illustrated in **FIG. 4****,** the lid 52 is moved down together with the operation pin 15 and is removed from the opening 59 of the container body 51. A plurality of cell culture containers 75 is taken out to the outside of the sealed container 50.

Next, a process of fixing the lid 52 to the container body 51 will be described.

First, as illustrated in **FIG. 3****,** the lid 52 is moved up together with the operation pin 15 and is inserted into the opening 59 of the container body 51. A plurality of cell culture containers 75 is accommodated within the sealed container 50. The gap between the lid 52 and the container body 51 is doubly sealed by the first seal element 53 and the second seal element 58. In the meantime, the gap between the inner peripheral portion that defines the second opening 52b of the lid 52 and the rotary member 55 is sealed by the second seal member 57.

Next, as illustrated in **FIG. 2****,** the operation pin 15 is rotationally driven by the motor 16. The rotary member 55 into which the tip portion of the operation pin 15 is fitted is rotated by the rotational power received from the operation pin 15. Since the operation pin 15 makes contact with the rotary member 55, the rotational power can be easily and reliably transmitted from the operation pin 15 to the rotary member 55.

The rotational motion of the rotary member 55 is converted to the linear motion of the locking members 54 by the conversion mechanism 56. Each of the locking members 54 is linearly moved in such a direction as to protrude from the first opening 52a. The tip portion of each of the locking members 54 is inserted into the locking hole 51a of the container body 51. Thus, the lid 52 is fixed to the container body 51. At this time, the inclined member 54a is pushed downward by the projection portion 52c. Thus, the tip portion of each of the locking members 54 is bent downward to push downward the inner surface that defines the locking hole 51a of the container body 51. By this push force, the lid 52 is pressed against the container body 51 and can be more firmly fixed to the container body 51.

According to the present embodiment described above, as the rotary member 55 is rotated, each of the locking members 54 is moved so as to protrude from the first opening 52a and the tip portion of each of the locking members 54 is inserted into the locking hole 51a of the container body 51. Thus, it is possible to fix the lid 52 without having to rotate the lid 52 with respect to the container body 51 and it is easy to automate the operation when attaching and detaching the lid. Furthermore, since the second seal member 57 is installed in the lid 52 so as to seal the second opening 52b, a gas existing outside the lid 52 is prevented from entering the interior of the sealed container 50 by sequentially passing through the second opening 52b and the first opening 52a. It is therefore possible to aseptically manage the interior of the sealed container 50.

When iPS cells are cultured, there is a possibility that the iPS cells are differentiated if stimulated by vibration or the like. However, according to the present embodiment, it is unnecessary to rotate the lid 52 relative to the container body 51 when attaching and detaching the lid 52. Thus, the vibration generated in the sealed container 50 when attaching and detaching the lid 52 is remarkably small. Accordingly, it is possible to accommodate vibration-sensitive cells such as iPS cells or the like at rest.

Furthermore, according to the present embodiment, the second seal member 57 is installed in the gap between the inner peripheral portion that defines the second opening 52b and the rotary member 55. At least a part of the end portion of the rotary member 55 opposite to the container body 51 is exposed to the outside. Thus, it is possible to bring the operation pin 15 existing outside the lid 52 into contact with the rotary member 55 while sealing the gap between the inner peripheral portion that defines the second opening 52b and the rotary member 55 with the second seal member 57. It is possible to easily and reliably transmit the rotational power from the operation pin 15 to the rotary member 55.

Furthermore, according to the present embodiment, the second seal element 58 is installed in the gap between the step portion 51b of the container body 51 and the edge of the container-body-side end portion of the lid 52. Thus, the gap between the lid 52 and the container body 51 is doubly sealed by the first seal element 53 and the second seal element 58 of the first seal member. Therefore, it is possible to reliably prevent a gas existing outside the lid 52 from entering the interior of the sealed container 50 through the gap between the lid 52 and the container body 51.

Furthermore, according to the present embodiment, the container body 51 is opened downward. Thus, as compared with a case where the container body 51 is laterally opened, it is possible to accommodate a plurality of stacked cell culture containers 75 with a smaller opening area. Moreover, it is possible to supply a plurality of cell culture containers to a culture device at a time. Therefore, it is possible to reduce the possibility that contaminants are taken into the sealed container 50.

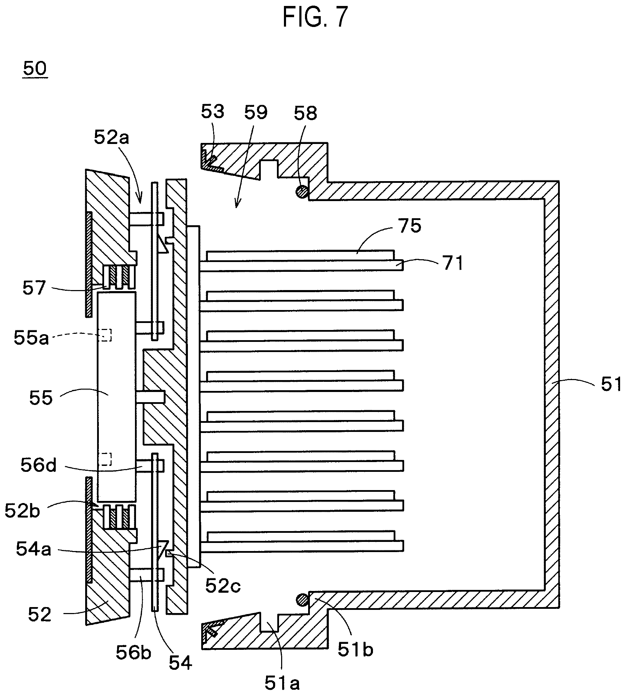

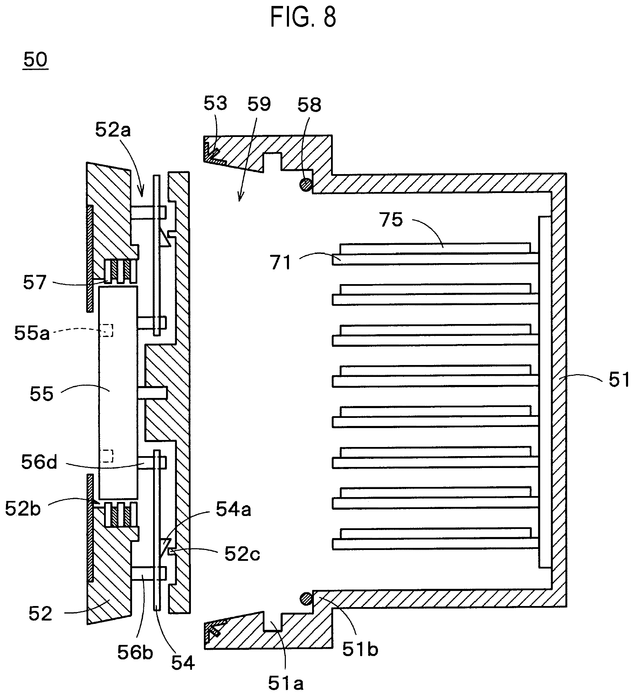

In the present embodiment, as illustrated in **FIGS. 1** to **4****,** the container body 51 is opened downward. However, the present disclosure is not limited thereto. For example, the container body 51 may be laterally opened as illustrated in **FIG. 7** or may be opened upward. Furthermore, as illustrated in **FIG. 8****,** the shelf 71 may be fixed to the container body 51 so that the cell culture containers 75 are taken out from the interior of the container body 51. In view of the convenience in accommodating a plurality of cell culture containers 75 in a stacked state, it is more preferable that the container body 51 is opened downward or upward.

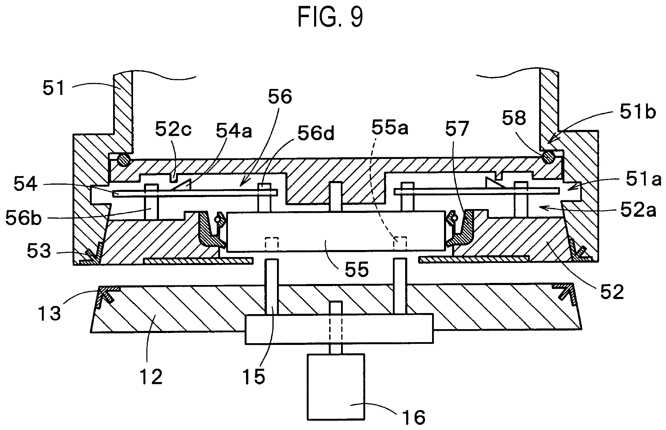

Furthermore, in the present embodiment, as illustrated in **FIGS. 1** to **4****,** the magnetic fluid seal is used as the second seal member 57. However, the present disclosure is not limited thereto. For example, as illustrated in **FIG. 9****,** an oil seal may be used as the second seal member 57. In the illustrated example, the second seal member 57 includes a ring-shaped lip member fixed to the inner peripheral portion that defines the second opening 52b, and oil filled in a gap between the lip member and the rotary member 55. The lip member is pressed against the rotary member 55 so that oil continues to be held in the gap between the lip member and the rotary member 55. Thus, while permitting rotation of the rotary member 55, it is possible to prevent a gas existing outside the lid 52 from entering the interior of the sealed container 50 through the second opening 52b. Alternatively, while not shown in the drawings, an O-ring seal or a bellows seal may be used as the second seal member 57. Even with such an aspect, the same action and effect as those of the present embodiment may be achieved.

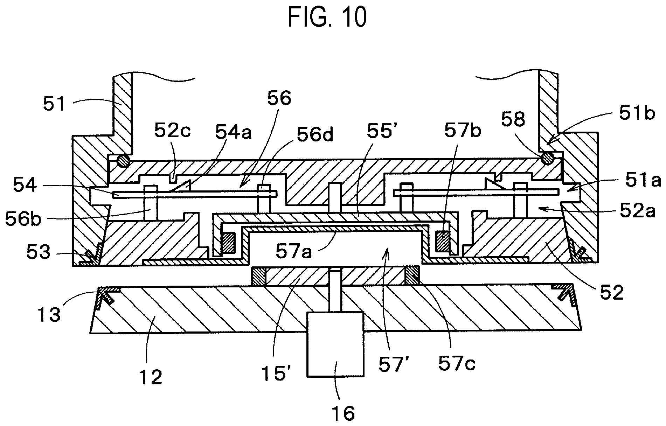

Moreover, in the present embodiment, as illustrated in **FIGS. 1** to **4****,** the second seal member 57 is installed in the gap between the inner peripheral portion that defines the second opening 52b of the lid 52 and the rotary member 55. At least a part of the end portion of the rotary member 55 opposite to the container body 51 is exposed to the outside. However, the present disclosure is not limited thereto. As illustrated in **FIG. 10****,** a second seal member 57' may be installed at the opposite side of a rotary member 55' from the container body 51 so as to cover the rotary member 55'. In this case, for example, a magnetic coupling seal may be used as the second seal member 57'.

More specifically, as illustrated in **FIG. 10****,** the second seal member 57' includes a partition wall 57a installed at the opposite side of a rotary member 55' from the container body 51 so as to cover the rotary member 55', and a driving magnet 57c and a driven magnet 57b disposed at the opposite sides of the partition wall 57a. The driven magnet 57b is fixed to the edge of the end portion of the rotary member 55' opposite to the container body 51. The driving magnet 57c is fixed to the edge of a disc member 15' connected to the motor 16. When the disc member 15' is positioned close to the partition wall 57a, the driving magnet 57c faces the driven magnet 57b via the partition wall 57a. A magnetic circuit is formed between the driving magnet 57c and the driven magnet 57b. If the disc member 15' is rotated together with the driving magnet 57c by the motor 16, the driven magnet 57b receives a rotational force applied by the magnetic force of the driving magnet 57c. Thus, the driven magnet 57b is rotated together with the rotary member 55. According to such an aspect, it is possible to more reliably prevent a gas existing outside the lid 52 from entering the interior of the sealed container 50 through the second opening 52b.

Furthermore, in the present embodiment, power is transmitted to the locking members 54 using the rotary member 55 and the conversion mechanism 56. However, the present disclosure is not limited thereto. For example, the locking members may be rotated without converting the rotational motion of the operation pin 15 to the linear motion of the locking members. Furthermore, for example, the back-and-forth motion caused by inserting the operation pin 15 in the front-rear direction may be converted to the up-down or left-right motion, thereby operating the locking members. In this case, the second seal member seals the gap between the member (movable member) that makes contact with the operation pin 15 and the lid 52. Furthermore, when the opening/closing operations are performed using a plurality of operation pins 15, a plurality of rotary members 55 may be installed.

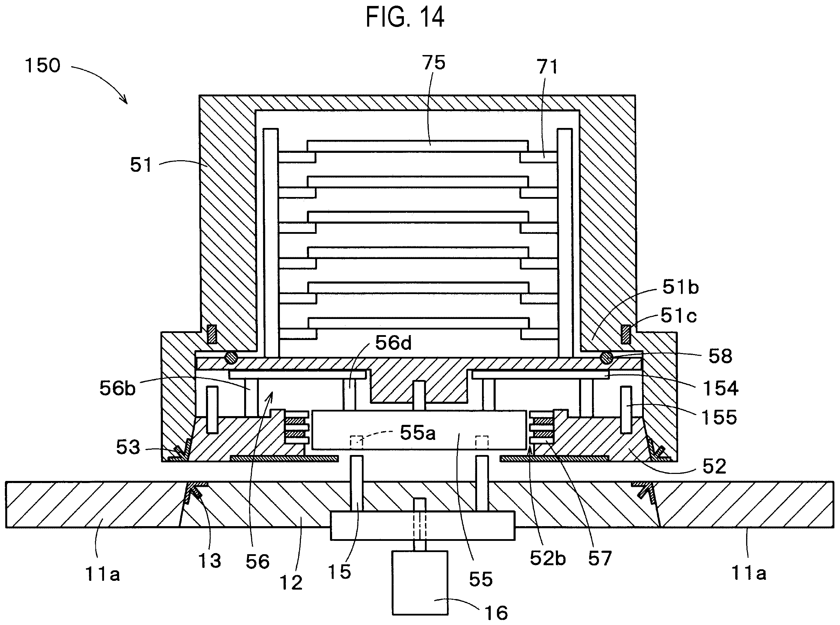

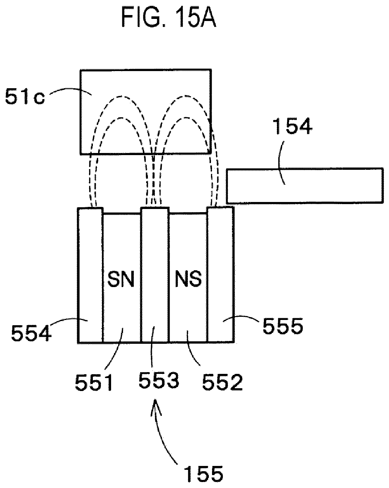

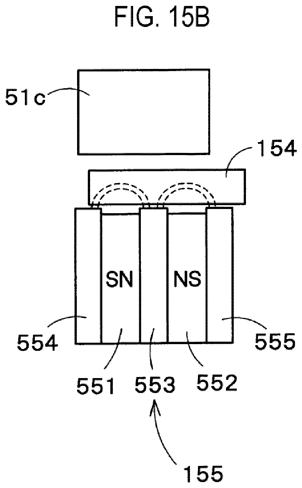

Next, a sealed container 150 according to a second embodiment of the present disclosure will be described with reference to **FIGS. 14****,** **15A** and **15B****.**

As illustrated in **FIG. 14****,** the sealed container 150 according to the second embodiment includes a metal body 51c, a magnet 155 and a yoke member 154, in place of the locking holes 51a and the locking members 54 of the first embodiment illustrated in **FIGS. 1** to **4****.**

As illustrated in **FIG. 14****,** the metal body 51c is installed in the step portion 51b of the container body 51.

In the meantime, the magnet 155 is installed within the lid 52 so as to correspond to the metal body 51c of the step portion 51b. The yoke member 154 is installed within the lid 52 so as to linearly move in a direction in which the yoke member 154 is inserted into or removed from a gap between the magnet 155 and the metal body 51c. The conversion mechanism 56 is configured to convert the rotational motion of the rotary member 55 to the linear motion of the yoke member 154.

**FIG. 15A** is a schematic view for explaining a magnetic circuit of a state in which the yoke member 154 is inserted into the gap between the magnet 155 and the metal body 51c. **FIG. 15B** is a schematic view for explaining a magnetic circuit of a state in which the yoke member 154 is removed from the gap between the magnet 155 and the metal body 51c.

In the present embodiment, as illustrated in **FIGS. 15A** and **15B****,** each magnet 155 includes a first magnet element 551 and a second magnet element 552, which have magnetic poles of the same polarity (N poles in the illustrated example) formed on the mutually-facing surfaces thereof. A first yoke element 553 is disposed between the first magnet element 551 and the second magnet element 552. Furthermore, a second yoke element 554 is disposed so as to face the surface of the first magnet element 551 opposite to the first yoke element 553 and a third yoke element 555 is disposed so as to face the surface of the second magnet element 552 opposite to the first yoke element 553.

As illustrated in FIG. **15A**, when the yoke member 154 is removed from the space between the magnet 155 and the metal body 51c, the magnetic force lines coming out from the N pole of the first magnet element 551 sequentially pass through the interior of the first yoke element 553, the interior of the metal body 51c and the interior of the second yoke element 554 and then enter the S pole of the first magnet element 551. Furthermore, the magnetic force lines coming out from the N pole of the second magnet element 552 sequentially pass through the interior of the first yoke element 553, the interior of the metal body 51c and the interior of the third yoke element 555 and then enter the S pole of the second magnet element 552. That is to say, a magnetic circuit is formed between the metal body 51c and the magnet 155. Thus, the magnetic body 211 is magnetized. By the magnetic force generated between the metal body 51c and the magnet 155, the lid 52 is fixed to the container body 51.

On the other hand, when the yoke member 154 is inserted into the space between the magnet 155 and the metal body 51c, the magnetic force lines coming out from the N pole of the first magnet element 551 sequentially pass through the interior of the first yoke element 553 and the interior of the yoke member 154. Thereafter, the magnetic force lines pass through the interior of the second yoke element 554 without passing through the interior of the metal body 51c and enter the S pole of the first magnet element 551. Furthermore, the magnetic force lines coming out from the N pole of the second magnet element 552 sequentially pass through the interior of the first yoke element 553 and the interior of the yoke member 154. Thereafter, the magnetic force lines pass through the interior of the third yoke element 555 without passing through the interior of the metal body 51c and enter the S pole of the second magnet element 552. That is to say, the magnetic circuit between the metal body 51c and the magnet 155 is interrupted by the yoke member 154. As a result, the magnetic force between the metal body 51c and the magnet 155 disappears, and the lid 52 can be removed from the container body 51.

In **FIGS. 14****,** **15A** and **15B****,** the same parts as those of the first embodiment illustrated in **FIGS. 1** to **4** are designated by the same reference numerals. Detailed descriptions thereof will be omitted.

Even with such a second embodiment, the same action and effect as in the first embodiment may be achieved.

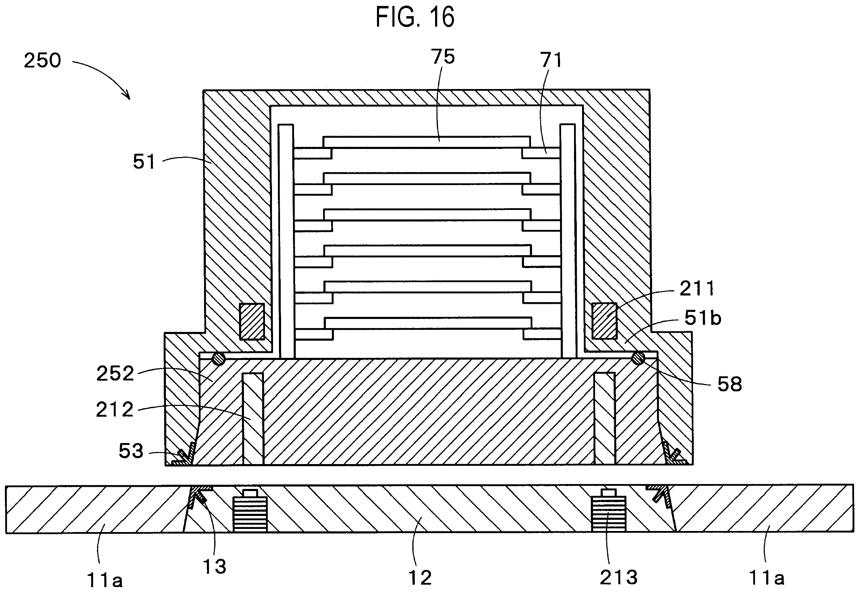

Next, a sealed container 250 according to a third embodiment of the present disclosure will be described with reference to **FIG. 16****.**

As illustrated in **FIG. 16****,** in the sealed container 250 according to the third embodiment, a permanent magnet 211 is installed in the step portion 51b of the container body 51. In the lid 252, a permanent electromagnet 212 that changes the magnetic force as a pulsed magnetic field is applied thereto is installed so as to correspond to the permanent magnet 211 of the step portion 51b.

The permanent electromagnet 212 is also called an electro-permanent magnet or a variable magnetic force magnet. Specifically, as the permanent electromagnet 212, for example, a samarium-cobalt magnet or an iron-cobalt-vanadium soft magnetic alloy is used.

In the present embodiment, an electromagnet 213 is installed outside the lid 252. In a state in which the electromagnet 213 is positioned in contact with or close to the end portion of the permanent electromagnet 212 opposite to the permanent magnet 211, a pulsed magnetic field is applied from the electromagnet 213 to the permanent electromagnet 212. Thus, the magnetic force of the permanent electromagnet 211 is changed according to the direction of the applied magnetic field.

More specifically, when a pulsed magnetic field of the same polarity as that of the permanent magnet 212 is applied from the electromagnet 213 to the permanent electromagnet 212, the magnetic force of the permanent electromagnet 212 is increased. On the other hand, when a pulsed magnetic field having a polarity opposite to the magnetic field of the permanent electromagnet 212 is applied from the electromagnet 213 to the permanent electromagnet 212, the magnetic force of the permanent electromagnet 212 is decreased. When a pulsed magnetic field having a polarity opposite to the magnetic field of the permanent electromagnet 212 is further applied from the electromagnet 213 to the permanent electromagnet 212, the direction of the magnetic force of the permanent electromagnet 212 is reversed.

In an initial state in which the lid 252 is fixed to the container body 51, the magnetic poles of opposite polarities are formed on the mutually-facing surfaces of the permanent magnet 211 and the permanent electromagnet 212. Due to the magnetic force (attractive force) generated between the permanent magnet 211 and the permanent electromagnet 212, the lid 252 is attracted to the container body 51.

In the initial state, if the electromagnet 213 is positioned in contact with or close to the end portion of the permanent electromagnet 212 opposite to the permanent magnet 211 and if a pulsed magnetic field having a polarity opposite to the magnetic field of the permanent electromagnet 212 is applied from the electromagnet 213 to the permanent electromagnet 212 to reverse the direction of the magnetic force of the permanent electromagnet 212, magnetic poles of the same polarity are formed on the mutually-facing surfaces of the permanent magnet 211 and the permanent electromagnet 212. By the magnetic force (repulsive force) generated between the permanent magnet 211 and the permanent electromagnet 212, the lid 252 can be removed from the container body 51 (first state).

Next, in the first state, if a pulsed magnetic field having a polarity opposite to the magnetic field of the permanent electromagnet 212 is applied from the electromagnet 213 to the permanent electromagnet 212 and if the direction of the magnetic force of the permanent electromagnet 212 is further reversed (namely, if the direction of the magnetic force of the permanent electromagnet 212 is returned to the direction of the magnetic force of the initial state), magnetic poles of opposite polarities are formed on the mutually-facing surfaces of the permanent magnet 211 and the permanent electromagnet 212. By the magnetic force (attractive force) generated between the permanent magnet 211 and the permanent electromagnet 212, the lid 252 is fixed again to the container body 51 (second state).

In this way, by applying a pulsed magnetic field from the electromagnet 213 to the permanent electromagnet 212 to change the direction of the magnetic force of the permanent electromagnet 212, the lid can be easily attached to and detached from the container body 51.

In **FIG. 16****,** the same parts as those of the first embodiment illustrated in **FIGS. 1** to **4** are designated by the same reference numerals. Detailed descriptions thereof will be omitted.

Even with such a third embodiment, the same action and effect as in the first embodiment may be achieved. In addition, since it is unnecessary to form the first opening 52a and the second opening 52b in the lid 252, it is possible to more reliably prevent a gas existing outside the lid 252 from entering the interior of the sealed container 50 through the interior of the lid 52.

In the third embodiment, the step portion 51b of the container body 51 may be provided with a magnetic body 211 such as iron or the like instead of the permanent magnet 211. In this case, in an initial state in which the lid 252 is fixed to the container body 51, the magnetic force lines coming out from the magnetic poles of the permanent electromagnet 212 pass through the magnetic body 211, whereby the magnetic body 211 is magnetized. The lid 252 is attracted to the container body 51 by the magnetic force (attractive force) generated between the magnetic body 211 and the permanent electromagnet 212.

In the initial state, if the electromagnet 213 is positioned in contact with or close to the end portion of the permanent electromagnet 212 opposite to the permanent magnet 211 and if a pulsed magnetic field having a polarity opposite to the magnetic field of the permanent electromagnet 212 is applied from the electromagnet 213 to the permanent electromagnet 212 so that the magnetic force of the permanent electromagnet 212 becomes zero (disappears), the magnetic force (attractive force) between the permanent magnet 211 and the permanent electromagnet 212 disappears and the lid 252 can be removed from the container body 51 (first state).

Next, in this first state, if a pulsed magnetic field is applied from the electromagnet 213 to the permanent electromagnet 212 and if the magnetic force of the permanent electromagnet 212 is restored, the magnetic force lines coming out from the magnetic poles of the permanent electromagnet 212 pass through the magnetic body 211, whereby the magnetic body 211 is again magnetized. The lid 252 is again fixed to the container body 51 by the magnetic force (attractive force) generated between the magnetic body 211 and the permanent electromagnet 212 (second state).

In this manner, by applying a pulsed magnetic field from the electromagnet 213 to the permanent electromagnet 212 to change the magnitude of the magnetic force of the permanent electromagnet 212, it is possible to easily attach and detach the lid 252 with respect to the container body 51.

Next, a conveyance system (a conveyance system according to an embodiment of the present disclosure) using the aforementioned sealed containers 50, 150 and 250 will be described. The conveyance system using the sealed container 50 of the first embodiment, the conveyance system using the sealed container 150 of the second embodiment and the conveyance system using the sealed container 250 of the third embodiment are the same as each other. Hereinafter, the conveyance system using the sealed container 50 of the first embodiment will be described as a representative example.

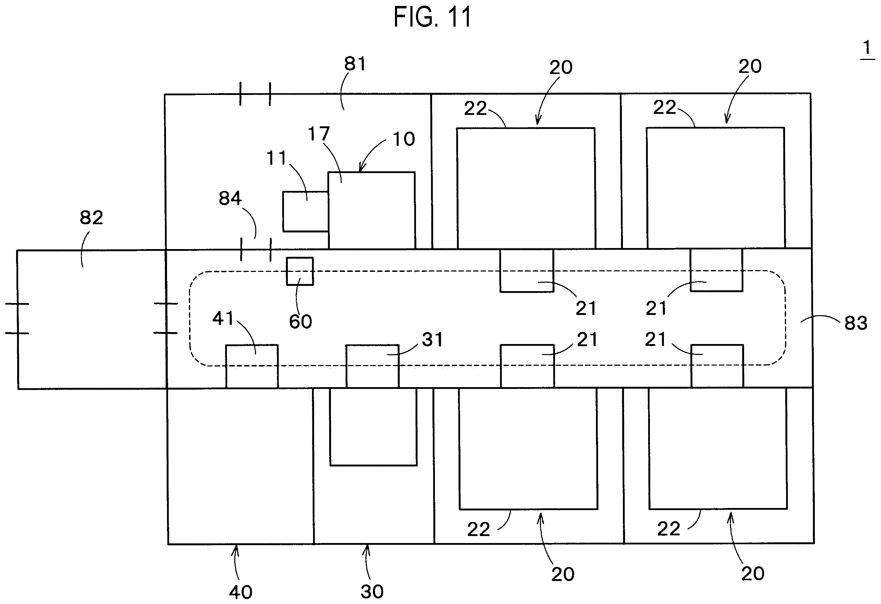

**FIG. 11** is a schematic plane view showing the configuration of the conveyance system 1 of the present embodiment.

As illustrated in **FIG. 11****,** the conveyance system 1 of this embodiment includes a conveyance part 60 configured to convey a sealed container 50 which accommodates cell culture containers or the like in a sealed state, and a plurality of (four, in the illustrated example) automatic culture devices 20 configured to take out cell culture containers from the sealed container 50 and to culture cells therein. The sealed container 50 is capable of holding a cell culture container on which cells to be cultured are mounted, an empty cell culture container, a cell culture container on which cultured cells are mounted, an unused material for use in cell culture, a used material for use in cell culture, and the like. In the present embodiment, as mentioned above, an aspect in which iPS cells are used will be described. Thus, the automatic culture devices 20 are iPS cell automatic culture devices for automatically culturing iPS cells. However, the automatic culture devices 20 may be differentiated cell automatic culture devices.

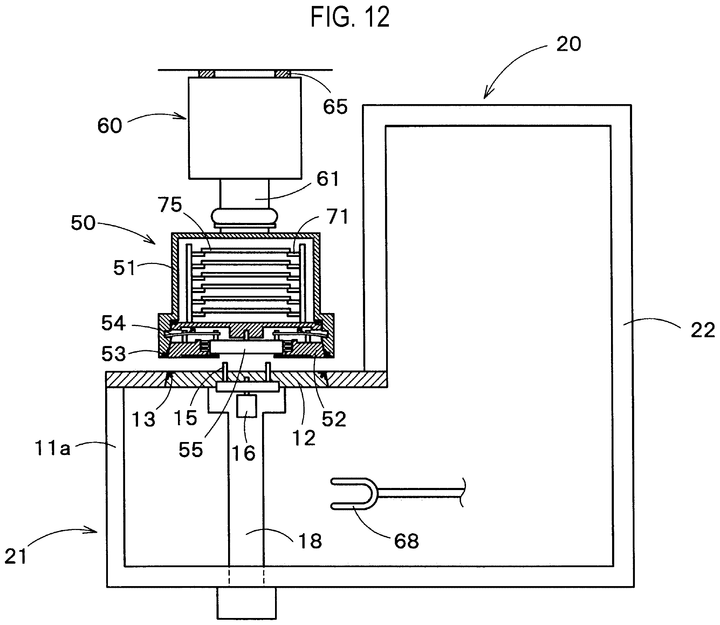

**FIG. 12** is a schematic side view illustrating, on an enlarged scale, the conveyance part 60 and the iPS cell automatic culture device 20. As illustrated in FIG. 12, the conveyance part 60 of the present embodiment includes a holding part 61 that holds the sealed container 50 so as to hang downward. The conveyance part 60 is configured to move along a rail 65 installed on the ceiling. Furthermore, the conveyance part 60 can vertically move the sealed container 50 held by the holding part 61.

In the meantime, the iPS cell automatic culture device 20 of the present embodiment includes a receiving part 21 installed below the rail 65 and configured to receive the sealed container 50 from the conveyance part 60, and a housing 22 connected to the receiving part 21. A conveyance arm 68 is installed within the housing 22.

As illustrated in **FIG. 12****,** the receiving part 21 includes a receiving part body 11a having an opening, a door 12 installed so as to be inserted into or removed from the opening of the receiving part body 11a, and a third seal member 13 installed in the receiving part body 11 a so as to seal a gap between the receiving part body 11a and the door 12.

In the present embodiment, as illustrated in **FIG. 12****,** the receiving part body 11a is opened upward. More specifically, in order to facilitate the insertion and removal of the door 12, the inner peripheral portion that defines the opening of the receiving part body 11a is formed so as to have a tapered shape whose opening area increases toward the lower side. Similarly, in order to facilitate the insertion and removal of the door 12, the side surface of the door 12 is formed so as to have a tapered shape whose cross-sectional area grows smaller toward the upper side.

As illustrated in **FIG. 12****,** the third seal member 13 of the present embodiment is formed of an elastic member (O-ring seal) having an annular plan-view shape and is tightly fixed to the edge of the upper end portion of the door 12. As illustrated in **FIG. 12****,** the third seal member 13 has a cross section of a substantially triangular shape (preferably, an obtuse-angled triangle shape). More specifically, the third seal member 13 has a first surface (outer circumferential surface) and a second surface (upper surface) inclined by a predetermined obtuse angle with respect to the first surface. The first surface of the third seal member 13 is positioned so as to protrude radially outward from the side surface of the door 12. The second surface of the third seal member 13 is positioned so as to protrude upward from the upper end portion of the door 12.

As illustrated in **FIG. 12****,** when the door 12 is inserted into the opening of the receiving part body 11a, the inner circumferential surface that defines the opening of the receiving part body 11a is brought into close contact with the first surface (outer circumferential surface) of the third seal member 13 without making contact with the side surface of the door 12. Thus, the gap between the door 12 and the receiving part body 11a is sealed by the third seal member 13.

In the present embodiment, as illustrated in **FIG. 12****,** an elevator device 18 is connected to the lower end portion of the door 12. The door 12 is supported at a desired height position by the elevator device 18. As the door 12 inserted into the opening of the receiving part body 11a is moved downward by the elevator device 18, the opening of the receiving part body 11a is opened.

Furthermore, in the present embodiment, when the sealed container 50 and the receiving part 21 are arranged in such an orientation that the respective openings face each other, the first seal element 53 of the first seal member is brought into close contact with the receiving part body 11a to seal the gap between the container body 51 and the receiving part body 11a. The third seal member 13 is brought into close contact with the lid 52 to seal the gap between the door 12 and the lid 52.

As illustrated in **FIG. 12****,** the operation pin 15 and the motor 16 described above are installed in the door 12 and are vertically moved together with the door 12 by the elevator device 18.

Next, with reference to **FIG. 12** and **FIGS. 1** to **4,** detailed descriptions will be made on a process in which the receiving part 21 of the iPS cell automatic culture device 20 receives the sealed container 50 from the conveyance part 60.

First, as illustrated in **FIG. 12****,** the sealed container 50 is conveyed by the conveyance part 60 and is positioned in a position where the opening of the sealed container 50 faces the opening of the receiving part 21. As illustrated in **FIG. 1****,** the lid 52 is inserted into the opening 59 of the container body 51, and the gap between the container body 51 and the lid 52 is sealed by the first seal element 53 and the second seal element 58 of the first seal member. Moreover, the door 12 is inserted into the opening of the receiving part body 11a. The gap between the receiving part body 11a and the door 12 is sealed by the third seal member 13.

Next, as illustrated in **FIG. 2****,** the sealed container 50 is moved vertically downward by the conveyance part 60. Thus, the bottom surface of the first seal element 53 is brought into close contact with the upper surface of the receiving part body 11a to seal the gap between the container body 51 and the receiving part body 11a. The upper surface of the third seal member 13 is brought into contact with the bottom surface of the lid 52 to seal the gap between the door 12 and the lid 52. At this time, the corner portion formed between the inner circumferential surface and the bottom surface of the first seal element 53 is brought into close contact with the corner portion formed between the outer circumferential surface and the upper surface of the third seal member 13.

Furthermore, as illustrated in **FIG. 2****,** the operation pin 15 installed in the door 12 is inserted from the second opening 52b of the lid 52. The tip portion of the operation pin 15 is fitted to the operation pin fitting hole 55a of the rotary member 55.

Next, as illustrated in **FIG. 3****,** the operation pin 15 is rotationally driven by the motor 16. The rotary member 55 is rotated by receiving the rotational power from the operation pin 15. The rotational motion of the rotary member 55 is converted to the linear motion of the locking members 54 by the conversion mechanism 56. The tip portion of each of the locking members 54 is removed from the locking hole 51a of the container body 51. Thus, the lid 52 can be removed from the container body 51.

Next, as illustrated in **FIG. 2****,** the door 12 is moved downward together with the lid 52 by the elevator device 18. Thus, as illustrated in **FIG. 12****,** the opening of the receiving part body 11a and the opening 59 of the container body 51 communicate with each other. At this time, since the gap between the container body 51 and the receiving part body 11a is sealed by the first seal element 53, dust, viruses and the like existing in the external space of the container body 51 and the receiving part body 11a are prevented from entering the internal space. Furthermore, since the gap between the door 12 and the lid 52 is sealed by the third seal member 13, dust, viruses and the like adhering to the upper end portion of the door 12 or the lower end portion of the lid 52 is prevented from entering the internal space of the container body 51 and the receiving part body 11a.

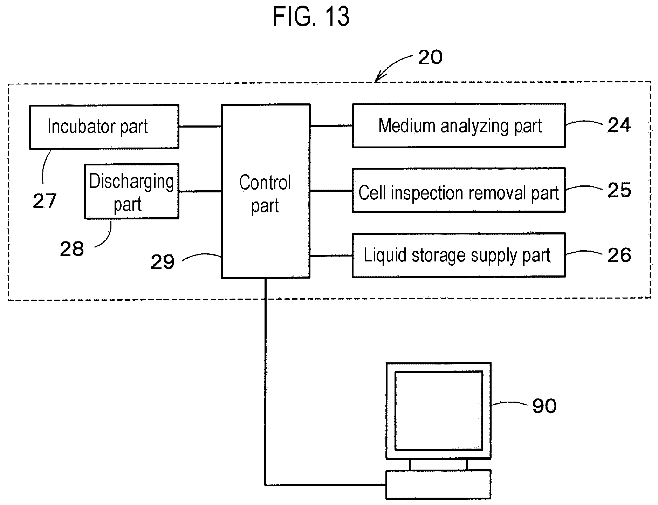

**FIG. 13** is a control block diagram illustrating a control mode of the iPS cell automatic culture device 20.

As illustrated in **FIG. 13****,** the iPS cell automatic culture device 20 includes a medium analyzing part 24 for analyzing a liquid medium component changing with the culture of iPS cells, a cell inspection removal part 25 for inspecting the iPS cells and removing the iPS cells with a poor condition, a liquid storage supply part 26 for storing and supplying a liquid medium or a liquid containing a proteolytic enzyme, an incubator part 27 for holding the cell culture containers 75 and automatically adjusting one, two or more of the temperature, the humidity and the gas concentration, and a discharging part 28 for discharging a waste liquid including a used liquid medium, a used cleaning liquid, a used reagent and the like, which are used within the iPS cell automatic culture device 20, downward from the housing 22.

Next, the operation of such an automatic iPS cell culture device 20 will be described.

First, the liquid storage supply part 26 automatically replaces the old liquid medium in the cell culture container 75 with a new liquid medium by appropriately supplying the liquid medium from the inlet port 75 a of the cell culture container 75 into the cell culture container 75. Based on the acquired iPS cell information, the cell inspection removal part 25 selectively separates defective iPS cells from an ECM (extracellular matrix) installed on the bottom surface of the cell culture container 75. Thereafter, the liquid storage supply part 26 pushes out the floating defective iPS cells from the cell culture container 75 by supplying the liquid medium into the cell culture container 75 from the inlet port 75a of the cell culture container 75. The liquid storage supply part 26 separates the iPS cells from the ECM installed on the bottom surface of the cell culture container 75, by appropriately supplying a proteolytic enzyme from the inlet port 75a of the cell culture container 75 into the cell culture container 75. Thereafter, the liquid storage supply part 26 pushes the floating iPS cells out of the cell culture container 75 by supplying the liquid medium from the inlet port 75a of the cell culture container 75 into the cell culture container 75. The pushed-out iPS cells are accommodated (seeded) in a plurality of separate cell culture containers 75 after they are diluted into a suspension. In this manner, the iPS cell automatic culture device 20 automatically performs subculture of the iPS cells. As the method for selectively separating the iPS cells contained in the cell culture container 75, it is possible to adopt a method of irradiating the iPS cells with ultrasonic waves or light, a method of applying a physical force from the outside of the cell culture container 75, or the like. In addition, when such a method is used, a proteolytic enzyme may be used in combination.

The internal temperature of the iPS cell automatic culture device 20 is adjusted by the incubator part 27 so as to become, for example, about 37 degrees C. Furthermore, the gas concentration within the iPS cell automatic culture device 20 is adjusted by appropriately adding CO₂ to the air with the incubator part 27. Moreover, if necessary, the humidity may be adjusted to become about 100% by the incubator part 27.

As illustrated in **FIG. 13****,** the iPS cell automatic culture device 20 includes a control part 29 communicatively connected to each of the medium analyzing part 24, the cell inspection removal part 25, the liquid storage supply part 26, the incubator part 27 and the discharging part 28 and configured to control them. Specifically, the control part 29 is installed in an external device 90 such as a personal computer or the like.

Referring back to **FIG. 11****,** the conveyance system 1 of the present embodiment further includes a sterilization device 10 configured to sterilize the interior of the sealed container 50. The sterilization device 10 includes a receiving part 11 installed in a receiving area 81 and configured to receive the sealed container 70 from a worker, and a sterilizing gas supply part 17 configured to sterilize the interior of the sealed container 50 received by the receiving part 11 by supplying a sterilizing gas such as a hydrogen peroxide gas or a high-temperature gas into the sealed container 50. As another example of the sterilization device 10, an aspect including an irradiation device configured to sterilize the interior of the sealed container 50 received by the receiving part 11 by irradiating gamma rays or ultraviolet rays into the sealed container 50 may be used instead of the sterilizing gas supply part 17. When the liquid medium or the like contains a protein or the like which may be damaged by gamma rays or ultraviolet rays, it is desirable to sterilize the liquid medium with a sterilizing gas such as a hydrogen peroxide gas or a high-temperature gas. The configuration of the receiving part 11 in the sterilization device 10 is substantially the same as the configuration of the receiving part 21 in the iPS cell automatic culture device 20 except that it is installed in the receiving area 81 to receive the sealed container 70 from a worker. Thus, detailed descriptions thereof will be omitted.

In the present embodiment, the receiving part 11 of the sterilization device 10 is installed in the receiving area 81 as shown in **FIG. 11****.** However, the present disclosure is not limited thereto. Just like the receiving part 21 of the iPS cell automatic culture device 20, the receiving part 11 of the sterilization device 10 may be installed below the rail 65 in a conveyance area 81. In this case, the receiving part 11 is configured to receive the sealed container 50 from the conveyance part 60.

The conveyance system 1 according to the present embodiment further includes an analysis device 30 configured to receive the cells cultured in the automatic culture device 20 at a predetermined timing and to inspect the cells, and a cryopreservation device 40 configured to freeze and store the iPS cells cultured in the automatic culture device 20.

As illustrated in **FIG. 11****,** each of the analysis device 30 and the cryopreservation device 40 includes a receiving part 31 or 41 installed below the rail 65 and configured to receive the sealed container 50 from the conveyance part 60. The configurations of the receiving part 31 of the analysis device 30 and the receiving part 41 of the cryopreservation device 40 are substantially the same as the configuration of the receiving part 21 of the automatic culture device 20 described above. Thus, detailed descriptions thereof will be omitted.

Next, the operation of the conveyance system 1 of the present embodiment will be described.

First, as illustrated in **FIG. 11****,** in the receiving area 81, one or two or more cell culture containers 75 containing iPS cells are accommodated in the sealed container 50 by, for example, a manual process performed by a worker.

Then, the sealed container 50 containing the cell culture container 75 is conveyed to the receiving part 11 of the sterilization device 10 by, for example, a manual process performed by a worker.

In the receiving part 21 of the sterilization device 10, the processes illustrated in **FIGS. 1** to **4****,** in which a conveyance process performed by the conveyance part 60 is replaced by a manual process performed by a worker, are performed in this order. Thus, while preventing entry of dust, viruses or the like into the internal spaces of the container body 51 and the receiving part body 11a, the opening of the receiving part body 11a and the opening of the container body 51 communicate with each other. Subsequently, the sterilizing gas is supplied from the sterilizing gas supply part 17 into the receiving part 11, and the interior of the receiving part body 11a and the container body 51 is sterilized.

Thereafter, the process illustrated in **FIGS. 1** to **4****,** in which a conveyance process performed by the conveyance part 60 is replaced by a manual process performed by a worker, is performed in the reverse order. Thus, while preventing entry of dust, viruses or the like into the internal spaces of the container body 51 and the receiving part body 11a, the opening of the container body 51 is sealed by the lid 52 and the first seal element 53, and the opening of the receiving part body 11a is sealed by the door 12 and the third seal member 13.

Next, as illustrated in **FIG. 11****,** the sealed container 50 remaining in a sealed state is placed on a loading part 84 communicating with the conveyance area 83 by, for example, a manual process performed by a worker. The loading part 84 is formed of, for example, dual doors. When the sealed container 70 is placed on the loading part 84, one of the doors positioned at the side of the receiving area 81 is opened (At this time, the door positioned at the side of the conveyance area 83 is kept closed). The sealed container 70 is introduced between the other door and the one door. The one door is closed. If necessary, the outer surface of the sealed container 50 is sterilized. Thereafter, the other door is opened. The sealed container 50 is received by the conveyance part 60 disposed in the conveyance area 83.

Subsequently, as illustrated in **FIG. 11****,** the sealed container 50 is conveyed to the receiving part 21 of the iPS cell automatic culture device 20 by the conveyance part 60.

In the receiving part 21 of the iPS cell automatic culture device 20, the processes illustrated in FIGS. 1 to 4 are performed in this order. Thus, while preventing entry of dust, viruses or the like into the internal spaces of the container body 51 and the receiving part body 11a, the opening of the receiving part body 11a and the opening of the container body 51 communicate with each other. Subsequently, the cell culture container 75 is taken out from the sealed container 50 to the iPS cell automatic culture device 20.

The iPS cells taken out to the iPS cell automatic culture device 20 in this way are cultured within the iPS cell automatic culture device 20. When the iPS cells are cultured, the replacement of the liquid medium is appropriately and automatically performed and the subculture of the iPS cells is appropriately and automatically performed. Furthermore, one, two or more of the temperature, the humidity and the gas concentration of the incubator part 27 may be adjusted. Incidentally, the iPS cell automatic culture devices 20 of the present embodiment are divided for each cell provider. When Mr. A's iPS cells are cultured in a certain iPS cell automatic culture device 20, iPS cells of a person other than Mr. A, an animal or the like are not cultured in the iPS cell automatic culture device 20.

Apart of the iPS cells cultured in the iPS cell automatic culture device 20 is appropriately taken out from the iPS cell automatic culture device 20 to the sealed container 50 and is then conveyed by the conveyance part 60 to the receiving part 31 of the iPS cell analysis device 30. Then, in the iPS cell analysis device 30, the state of culture (for example, the state of DNA) is analyzed. The inspection performed in the iPS cell analysis device 30 is usually a destructive inspection differing from the inspection performed in the iPS cell automatic culture device 20. The iPS cells used for the analysis are discarded without being returned to the iPS cell automatic culture device 20.

When the iPS cells are cultured in the iPS cell automatic culture device 20, one or two or more cell culture containers 75 containing the iPS cells are placed on a plurality of shelves 71 supported on the lid 52 of the sealed container 50. Subsequently, the processes illustrated in **FIGS. 1** to **4** are performed in the reverse order. Thus, while preventing entry of dust, viruses or the like into the internal spaces of the container body 51 and the receiving part body 11a, the opening of the container body 51 is sealed by the lid 52 and the first seal element 53, and the opening of the receiving part body 11a is sealed by the door 12 and the third seal member 13. The sealed container 50 remaining in a sealed state is received by the conveyance part 60 and is suspended.

Next, when the cultured iPS cells are frozen and stored, the sealed container 50 containing the cultured iPS cells is conveyed to the receiving part 41 of the cryopreservation device 40 by the conveyance part 60. Then, in the cryopreservation device 40, the cell culture container 75 is taken out from the interior of the sealed container 50. The iPS cells are preserved in a state in which the iPS cells are accommodated in the cell culture container 75 within the cryopreservation device 40.

When the iPS cells preserved in the cryopreservation device 40 are shipped, the cell culture container 75 containing the iPS cells is accommodated in the sealed container 50. The sealed container 50 is conveyed to a shipping area 82 by the conveyance part 60. When the sealed container 50 is conveyed to the shipping area 82, the iPS cells are shipped in the form of the sealed container 50 or the cell culture container 75 taken out from the sealed container 50.

Incidentally, it is not necessary to cryopreserve the iPS cells when a shipping destination (for example, a hospital, a factory, etc.) of the iPS cells is located adjacent to the above-described conveyance system 1 or in the neighborhood thereof. Therefore, in this case, the cell culture container 75 containing the iPS cells and taken out from the iPS cell automatic culture device 20, is accommodated in the sealed container 50 and is then conveyed to the shipping area 82 by the conveyance part 60 without being cryopreserved. Thereafter, the iPS cells are shipped from the shipping area to the neighboring or adjacent shipping destination in the form of the sealed container 50 or the cell culture container 75 taken out from the sealed container 50.

In the conveyance system 1 of the present embodiment, the interior of the sealed container 50 and the interior of the iPS cell automatic culture device 20 are managed at a relatively high cleanliness. By doing so, it is possible to manage the cleanliness at a high value only in the space having a high influence on the cell culture. Thus, the management cost can be kept low. In addition, since each of the spaces is partitioned into comparatively small spaces, it is also possible to isolate only the space requiring a sterilization work and to perform a sterilization work. It is possible to operate the conveyance system 1 with good maintainability.

In addition, the receiving area 81 and the shipping area 82 where workers enter and exit have a relatively lower cleanliness than the conveyance area 83. However, by using the sealed container 50 of the present embodiment, it is possible to handle the cells while keeping the cleanliness.

Following the culture of the iPS cells, cells differentiated into arbitrary cells may be cultured using the same device.

The term "aseptic" used herein does not refer to only the state in which microorganisms are completely sterilized but refers to the state in which the number of microorganisms which may adversely affect the culture of cells is controlled to an appropriate number or less.

In the above-described embodiment, the sealed container 50, 150 or 250 is used to accommodate a cell culture container or the like for culturing cells in a sealed state. However, the present disclosure is not limited thereto. The sealed container 50, 150 or 250 may be used to accommodate a semiconductor wafer in a sealed state.

### EXPLANATION OF REFERENCE NUMERALS

1: conveyance system, 10: sterilization device, 11: receiving part, 11a: receiving part body, 12: door, 13: third seal member, 15: operation pin, 16: motor, 17: sterilizing gas supply part, 18: elevator device, 20: automatic culture device, 21: receiving part, 22: housing, 24: medium analyzing part, 25: cell inspection removal part, 26: liquid storage supply part, 27: incubator part, 28: discharging part, 29: control part, 30: analysis device, 31: receiving part, 40: cryopreservation device, 41: receiving part, 50: sealed container, 150: sealed container, 250: sealed container, 51: container body, 51a: locking hole, 51b: step portion, 51 c: metal body, 52: lid, 52a: first opening, 52b: second opening, 53: first seal element, 53a: first surface, 53b: second surface, 54: locking member, 55: rotary member, 55': rotary member, 56: conversion mechanism, 57: second seal member, 57': second seal member, 57a: partition wall, 57b: driving magnet, 57c: driven magnet, 57d: seal cover, 58: second seal element, 59: opening, 60: conveyance part, 61: holding part, 65: rail, 68: conveyance arm, 71: shelf, 75: cell culture container, 75a: inlet port, 75b: passage, 75c: outlet port, 81: receiving area, 82: shipping area, 83: conveyance area, 84: loading part, 90: external device, 154: yoke member, 155: magnet, 211: permanent magnet, 212: permanent electromagnet, 213: electromagnet, 551: first magnet element, 552: second magnet element, 553: first yoke element, 554: second yoke element, 555: third yoke element

## Claims

1. A sealed container, comprising:
a container body having an opening;
a lid removably installed in the opening of the container body; and
a first seal member installed so as to seal a gap between the container body and the lid,
wherein a locking hole is formed in an inner peripheral portion that defines the opening of the container body,
a first opening capable of facing the locking hole is formed in an outer peripheral portion of the lid,
a second opening communicating with the first opening is formed in an end portion of the lid opposite to the container body, and
the lid includes a locking member capable of linearly moving in such a direction as to protrude from and retract into the first opening, a rotatable rotary member positioned to face the second opening, a conversion mechanism configured to covert rotational motion of the rotary member to linear motion of the locking member, and a second seal member installed so as to seal the second opening.

2. The container of Claim 1, wherein the second seal member is installed in a gap between an inner peripheral portion that defines the second opening and the rotary member, and
at least a part of an end portion of the rotary member opposite to the container body is exposed to the outside.

3. The container of Claim 2, wherein the second seal member is one of a magnetic fluid seal, an oil seal, an O-ring seal and a bellows seal.

4. The container of Claim 1, wherein the second seal member is installed at an opposite side of the rotary member from the container body so as to cover the rotary member.

5. The container of Claim 4, wherein the second seal member is a magnetic coupling seal.

6. A sealed container, comprising:
a container body having an opening;
a lid removably installed in the opening of the container body; and
a first seal member installed so as to seal a gap between the container body and the lid,
wherein the container body includes a step portion capable of facing an edge of a container-body-side end portion of the lid and a metal body installed in the step portion,
a second opening is formed in an end portion of the lid opposite to the container body, and
the lid includes a magnet installed so as to correspond to the metal body of the step portion, a yoke member capable of linearly moving in such a direction that the yoke member is inserted into and removed from between the magnet and the metal body, a rotatable rotary member positioned to face the second opening, a conversion mechanism configured to convert rotational motion of the rotary member to linear motion of the yoke member, and a second seal member installed so as to seal the second opening.

7. A sealed container, comprising:
a container body having an opening;
a lid removably installed in the opening of the container body; and
a first seal member installed so as to seal a gap between the container body and the lid,
wherein the container body includes a step portion capable of facing an edge of a container-body-side end portion of the lid and a permanent magnet or a magnetic body installed in the step portion, and
a permanent electromagnet configured to change a magnetic force as a pulsed magnetic field is applied thereto is installed in the lid so as to correspond to the permanent magnet or the magnetic body of the step portion.

8. The container of Claim 7, wherein the permanent electromagnet is a samarium-cobalt magnet or an iron-cobalt-vanadium soft magnetic alloy.

9. A sealed container, comprising:
a container body having an opening;
a lid removably installed in the opening of the container body; and
a first seal member installed so as to seal a gap between the container body and the lid,
wherein a locking member configured to lock the lid to the container body, a movable member configured to move the locking member, and a second seal member configured to seal a gap between the lid and the movable member, are installed within the lid.

10. The container of any one of Claims 1 to 9, wherein the first seal member includes a first seal element installed in an edge of an inner peripheral portion that defines the opening of the container body.

11. The container of any one of Claims 1 to 10, wherein the first seal member includes a second seal element installed in a gap between a step portion capable of facing an edge of a container-body-side end portion of the lid and the edge of the container-body-side end portion of the lid.

12. The container of Claim 10, wherein the first seal element is an O-ring seal having a substantially triangular cross section.

13. The container of any one of Claims 1 to 12, wherein a shelf capable of accommodating a plurality of cell culture containers is installed inside the container body.

14. A conveyance system, comprising:
the sealed container of any one of Claims 1 to 13;
a conveyance part configured to convey the sealed container; and
a receiving part configured to receive the sealed container conveyed by the conveyance part,
wherein the receiving part includes a receiving part body having an opening, a door removably installed in the opening of the receiving part body, and a third seal member installed in an edge of the door so as to seal a gap between the receiving part body and the door,
the first seal member includes a first seal element installed in an edge of an inner peripheral portion that defines the opening of the container body, and
when the sealed container and the receiving part are disposed in such an orientation that the openings of the sealed container and the receiving part face each other, the first seal element is brought into close contact with the receiving part body to seal a gap between the container body and the receiving part body, and the third seal member is brought into close contact with the lid to seal a gap between the door and the lid.
